Europäisches Patentamt

European Patent Office

Office européen des brevets

(11) Publication number: **0 302 429 B1**

# EUROPEAN PATENT SPECIFICATION

(45) Date of publication of patent specification: **28.07.93**  (51) Int. Cl.⁵: **C12P 21/02**, C12N 15/24

(21) Application number: **88112458.0**

(22) Date of filing: **01.08.88**

(54) **Novel human interleukin 4, expression vectors for human interleukin 4 and transformants containing the same.**

(30) Priority: **03.08.87 JP 192666/87**
**24.02.88 JP 39482/88**

(43) Date of publication of application:
**08.02.89 Bulletin 89/06**

(45) Publication of the grant of the patent:
**28.07.93 Bulletin 93/30**

(84) Designated Contracting States:
**AT BE CH DE ES FR GB GR IT LI LU NL SE**

(56) References cited:
**EP-A- 0 230 107**
**EP-A- 0 254 399**

**THE JOURNAL OF BIOCHEMISTRY, vol. 102, no. 1, July 1987, pages 123-131, Tokyo, JP; K. ONOMICHI et al.: "Expression of amplified cDNA and genomic sequences encoding human interleukin 2 in chinese hamster ovary cells"**

**JOURNAL OF EXPERIMENTAL MEDICINE, vol. 163, June 1986, pages 1405-1414, The Rockefeller University Press; K. GRABSTEIN et al.: "Purification to homogeneity of B cell stimulating factor"**

(73) Proprietor: **Honjo, Tasuku**
**Higashi-1-jo-agaru, Nishi-iru, Izumidono-cho**
**Yoshida, Sakyo-ku, Kyoto-shi 606(JP)**

Proprietor: **ONO PHARMACEUTICAL CO., LTD.**
**No. 14, Doshomachi 2-chome Higashi-ku**
**Osaka-shi Osaka(JP)**

(72) Inventor: **Honjo, Tasuku,**
**Higashi-1-jo-agaru, Nishi-iru, Izumidono-cho**
**Yoshida, Sakyo-ku, Kyoto-shi 606(JP)**
Inventor: **Hama, Kazuaki,**
**169, Hamadera-Suwanomori-higashi 2-cho**
**Sakai-shi, Osaka,(JP)**
Inventor: **Kawasaki, Akiyoshi,**
**8-5, Sakurai 4-chome, Shimamoto-cho,**
**Mishima-gun**
**Osaka(JP)**

(74) Representative: **Henkel, Feiler, Hänzel & Partner**
**Möhlstrasse 37**
**W-8000 München 80 (DE)**

FEDERATION PROCEEDINGS, vol. 46, no. 6, 1987, page 2230, no. 1775; H.V. LE et al.: "Characterization of recombinant human interleukin 4 derived from COS-7 monkey kidney cells"

**Description**

FIELD OF THE INVENTION

The present invention relates to novel human IL4 having immune-activating effects, novel expression vectors for said human IL4 and novel transformants thereof.

BACKGROUND OF THE INVENTION

Interleukin 4 (hereafter "IL4") is a glycoprotein produced by T lymphocytes when stimulated with lectin, phorbol ester or antigen. IL4 has various significant effects on the immune response, such as differentiation and proliferation, of B lymphocytes, of T lymphocytes and of mast cells (see Y. Noma et al., Nature, 319, 640-646, 1986 (hereafter "Reference A"); F. Lee et al., Proc. Natl. Acad. Sci. USA, 83, 2061-2065, 1986 (hereafter "Reference B"); E. Severinson et al., Eur. J. Immunol., 17, 67-72, 1987 and T. R. Mosmann et al., Proc. Natl. Acad. Sci. USA, 83, 5654-5658, 1986). Since IL4 has such strong immune-activating effects, it is believed that IL4 is useful for the prevention and treatment of infections, AIDS, cancer, immune deficiency, etc. (see R. Fernandez-Botran et al., Proc. Natl. Acad. Sci. USA, 83, 9689-9693, 1986 and R. Palacios et al., EMBO J., 6, 91-95, 1987).

Native human IL4 can be obtained by treating normal human peripheral blood lymphocytes, malignant human T cell lines or human T cell hybridoma with drugs. However, since only a very small amount of IL4 is produced by these methods, physical and chemical characteristics (e.g. the total molecular weight, molecular weight of the sugar chain, the isoelectric point, etc.) of human IL4 *per se* have not yet been determined. Further, it has been impossible to obtain a sufficant amount of purified human IL4 for clinical use. Hence, it has been desired to develop a method for preparing large amounts of IL4 by utilizing genetic engineering techniques.

In recent years, the cDNA of mouse IL4 has been cloned (see Reference A and Reference B). Further the cDNA of human IL4 has been cloned by using the mouse DNA as probe (see T. Yokota et al., Proc. Natl. Acad. Sci. USA, 83, 5894-5898, 1986 (hereafter "Reference C")). The nucleotide sequence thereof and the expression of human IL4 by culturing monkey cells transfected with the human cDNA, have been reported (see Reference C and PCT Patent International publication No. WO 87/02990). Further, a yeast expression system for producing human IL4 has been reported (see J. Exp. Med., 166, 476, 1987). However, IL4 is expressed in monkey cells transiently, and the cells die after producing human IL4. Thus, the cells used are not available for expression again, and it is necessary to always transfect fresh cells. Moreover, a number of DNA vector and a good deal of work are required for transfection. Thus, the method using monkey cells is not suitable for supplying enough human IL4 for clinical use. To date, there has been no report of an effective expression system for human IL4.

In order to obtain foreign gene products utilizing genetic engineering techniques, many expression vectors and hosts for expressing the vector (for example, procaryotic cells such as Escherichia coli, Bacillus subtilis, Actinomycetes, Lactobacillus, etc. and eucaryotic cells, such as yeast, fungi, mammalian cells, etc.) have ever been found. Recently, it has been observed that it is necessary to select an expression vector and a host most suitable for the gene in order to obtain a particular gene product, especially with high efficiency as used in commercial production.

SUMMARY OF THE INVENTION

Accordingly, an object of the present invention is to provide large quantities of human IL4 useful for the prevention and treatment of infections, AIDS, cancer, immunodeficiency, etc.

Another object of the present invention is to provide an expression vector for the production of large quantities of human IL4.

Still another object of the present invention is to provide transformants which express large quantities of human IL4.

The above-described objects of the present invention have been met by a novel human IL4 having specific physical and chemical characteristics produced by utilizing an expression vector which comprises a DNA sequence encoding human IL4 at a position downstream from an SV40 early promoter, and a DNA sequence encoding dihidrofolate reductase (hereafter "DHFR") at a position downstream from the promoter, and by utilizing dihydrofolate reductase-defective Chinese hamster ovary cells (hereafter "CHO cells"), as host cells.

BRIEF DESCRIPTION OF THE DRAWINGS

Figure 1 shows the nucleotide sequence of the cDNA of human IL4 and the amino acid sequence deduced therefrom.

Figure 2 shows construction of the pKCRhIL4-Dn expression vector for human IL4.

Figure 3 shows construction of the pKCRhIL4Δ-D expression vector for human IL4.

Figure 4 is a densitometer graph obtained after separating human IL4 by SDS/PAGE.

DETAILED DESCRIPTION OF THE INVENTION

As a result of continuous research on an IL4 expression system using a mammalian cells as host, an expression system with high efficiency has been found in the present invention comprising the combination of an expression vector containing SV40 early promoter and CHO cells. It has also been found in the present invention that by incorporating the gene of DHFR in the expression vector and further by using DHFR-defective cells, an expression system with more high efficiency can be obtained.

Furthermore, it has been confirmed that novel human IL4 produced by the expression system of the present invention has specific physical and chemical characteristics (e.g. molecular weight of the sugar chain, i.e. the sugar content of the human IL4, etc.)

Although an expression system using CHO cells as host has been already applied to the expression of some biological active proteins, each protein has quite different sugar chain, length of the chain, sugar components, etc. These differences are represented by the molecular weight of the sugar chain, etc, as shown in Table I below.

Table I

| Proteins | molecular weight of sugar chain (Kd) |
|---|---|
| Interferon-$\gamma$[1] | 5 |
| Erythropoietin[2] | 15 |
| Tissue plasminogen activator (t-PA)[3] | 10 |

1) Roger Dijkmans et al., J. Biol. Chem., 262, 2528-2535, 1987
2) Fu-Kuen Lin et al., Proc. Natl. Acad. Sci. USA, 82, 7580-7584, 1985
3) Randal J. Kaufman et al., Molecular and Cellular Biology, 5, 1750-1759, 1985

Accordingly, it can not be said that glycoproteins having sugar components with the same characteristics are produced, when the expression system using CHO cells is applied to the expression of various glycoproteins.

The present invention provides in particular:

(1) novel human IL4 characterized in that:
   (a) the total molecular weight is 17000 to 19000, and
   (b) the molecular weight of the sugar content is 2000 to 4000;
(2) a novel expression vector for the human IL-4 of (1) which comprises:
   (i) a DNA sequence encoding human IL4 at a position downstream from an SV40 early promoter, and
   (ii) a DNA sequence encoding DHFR at a position downstream from a promoter operating in eukaryotic cells;
(3) a novel transformant which is obtained by transfecting DHFR-defective CHO cells with the vector of (2); and
(4) a novel human IL4 which is produced by using the transformant of (3) as the host.

In human IL4, the amino acid sequence of the protein moiety thereof is shown by the following formula:

```
MetGlyLeuThrSerGlnLeuLeuProProLeuPhePheLeu

LeuAlaCysAlaGlyAsnPheValHisGlyHisLysCysAsp

IleThrLeuGlnGluIleIleLysThrLeuAsnSerLeuThr

GluGlnLysThrLeuCysThrGluLeuThrValThrAspIle

PheAlaAlaSerLysAsnThrThrGluLysGluThrPheCys

ArgAlaAlaThrValLeuArgGlnPheTyrSerHisHisGlu

LysAspThrArgCysLeuGlyAlaThrAlaGlnGlnPheHis

ArgHisLysGlnLeuIleArgPheLeuLysArgLeuAspArg

AsnLeuTrpGlyLeuAlaGlyLeuAsnSerCysProValLys

GluAlaAsnGlnSerThrLeuGluAsnPheLeuGluArgLeu

LysThrIleMetArgGluLysTyrSerLysCysSerSer
```

(see Reference C).

It has been confirmed that the human IL4 of the present invention contains 129 amino acids from the 25th amino acid, histidine to the 153rd amino acid, serine, and lacks amino acids from the first amino acid, methionine to the 24th amino acid, glycine (see Examples 8 and 9 described hereinafter).

The sugar content of the human IL4 of the present invention is quite distinct from that of the human IL4 known to date.

The molecular weight of the human IL4 of the present invention is 17 to 19 kd (kilodaltons). The molecular weight of only the protein moiety is 14962. Therefore, the difference in molecular weight between total human IL4 and the protein moiety, i.e. 2000 to 4000, preferably 2500 to 3500, is considered to be the molecular weight of the sugar content.

On the other hand, the molecular weight of human IL4 expressed by monkey cells, described in PCT Patent International Publication No. WO 87/02990 is 22 kd, and hence that of the sugar content is considered to be about 7 kd. Therefore, it is confirmed that the quantity of the sugar content bonded to the protein moiety in the human IL4 expressed by monkey cells is more than two times that in the human IL4 of the present invention. In addition, there is a great difference in molecular weight between the sugar content of both human IL4s.

Furthermore, the molecular weight of human IL4 expressed by yeast is 60 kd and that of the sugar content is considered to be 45 kd. Hence, the molecular weight of the sugar content of the human IL4 expressed by yeast is greatly different from that of the present invention.

The human IL4 of the present invention preferably has a isoelectric point of 8.5 to 10.5, more preferably 9.8 to 10.1.

More preferably, the human IL4 of the present invention has a sugar content which comprises at least mannose, fucose and galactose as neutral sugars, at least glucosamine as an amino sugar and at least N-acetylneuraminic acid as a sialic acid.

Further, the human IL4 of the present invention preferably has a sugar content wherein mannose, fucose, galactose, glucosamine and N-acetylneuraminic acid are contained in amounts of 3.0 to 4.0, 1.0 to 2.0, 2.5 to 3.5, 4.5 to 5.5 and 1.5 to 2.5, mols per total mol of sugar, respectively.

The human IL4 of the present invention is sufficiently pure, but is not necessarily a single chemical compound. The present invention may comprise many homogenous human IL4 having various characteristics within the scope described above and heterogeneous human IL4 having slightly different sugar contents.

The present invention also includes the expression vector for human IL4 and the transformant.

The expression vector for human IL4, of the present invention may be prepared by ligating:

(iii) a DNA sequence containing an origin of replication and drug resistance gene in Escherichia coli, besides the DNA sequences of (i) and (ii) mentioned above.

The DNA of human IL4 may be prepared by (1) extracting mRNA from cells being capable of producing IL4, such as normal human lymphycytes, (2) constructing cDNA by using reverse transcriptase, and, then, (3) cloning the cDNA from the obtained cDNA library in Escherichia coli. For example, the desired cDNA may be selected from a cDNA library by cross-hybridization using a part of the cDNA of mouse IL4 (described in Reference A and B) as a probe or by using the biological activities of human IL4 as a screen. Efficient selection is a obtained by using both of the above methods together. It is also possible to select by hybridization using a synthetic oligonucleotide having a partial nucleotide sequence of cDNA of human IL4, which has already reported (described in Reference C), as probe.

Recently, it has been reported that a nucleotide sequence rich in adenine (A) and uracil (U) exists in a non-translated region at the 3′-side of mRNA of lymphokines such as IL4 and that this sequence relates to the instability of mRNA itself (see G. Shaw et al., Cell, 46, 659-667, 1986). It is considered by the present inventors that in IL4, three groups of the nucleotide sequence shown by "ATTTA" (i.e., from 472nd to 476th, from 506th to 510th and from 514th to 518th), correspond to that relating to instability. Therefore, if the desired products are considered to be produced more efficiently, host cells are transfected with an IL4 vector wherein the nucleotide sequence for instability has been removed. As a result of a study based on these hypotheses, it has been found in the present invention that by removing the entire nucleotide sequence existing in the position downstream from the nucleotide sequence signaling for the termination of translation of human IL4 (i.e., the nucleotide sequence downstream adenine, the 464th), human IL4 is produced several ten times more, as compared with the use of a vector wherein this sequence is not removed. It is the present inventors who have first confirmed such fact by experiments. Of course, the production of IL4 is considered to increase similarly by removing only three groups of the nucleotide sequence for instability.

The DNA of human IL4 wherein the nucleotide sequence for instability is removed may be prepared by (1) cleaving the vector DNA containing the cDNA of human IL4 by a restriction enzyme which recognizes a particular nucleotide sequence existing singly at a position downstream from the nucleotide sequence for instability, e.g. Sal I, (2) digesting with a nuclease such as Bal 31, and ,then, (3) cloning the obtained DNA fragment into Escherichia coli.

By using either the DNA wherein at least the nucleotide sequence for instability is or is not removed, the purpose of the present invention may be accomplished. The expression "the DNA where at least the nucleotide sequence for instability is removed" means that three groups of nucleotide sequences shown by "ATTTA" in the 3′ non-translated region of the DNA are removed alone or along with some or all of the remaining 3′ non-translated region. However, it is preferable to use the DNA wherein at least the nucleotide sequence for instability is removed.

As vectors for expressing human IL4 in a mammalian cell, any vectors containing an SV40 early promoter may be used. Examples of such vecters are pSV2 vector (see R. C. Mulligan et al., Science, 209, 1422-1427, 1980) and pKCR•H$_2$ vector (see K. O'Hare et al., Proc. Natl. Acad. Sci., USA, 78, 1527-1531, 1981). pKCR•H$_2$ vector is preferred.

pKCR•H$_2$ vector has a splicing sequence and polyadenylation sequence, both derived from the gene of $\beta$-globin, besides an SV40 early promoter. Therefore, it is suitable for the expression of the desired product in mammalian cells. Furthermore, this vector has an origin of replication in Escherichia coli and an ampicillin resistance gene as a selection marker, in order to enable the replication in Escherichia coli. Hence, pure vector DNA may be prepared in quantity by using Escherichia Coli.

The DHFR gene may be prepared by the method known (see R. J. Kaufman et al., Molecular and Cellular Biology, 2, 1304-1319, 1982). As promoters for the expression of the DHFR gene, any promoters operating in eukaryotic cells, such as an SV40 early promoter, metallothionein gene promoter, adenovirus gene promoter, retrovirus gene promoter etc. may be used.

By combining three DNA sequences having above-mentioned features, the expression vector for human IL4 may be prepared, and the preparation may be carried out by conventional methods well-known to those skilled in the art (see T. Maniatis et al., Molecular Cloning, Cold Spring Harbor Laboratory, 1982).

Dihydrofolate reductase-defective Chinese hamster ovary cells (hereafter "CHO dhfr− cells") for the transfection of the expression vector for human IL4 are described in G. Urlaub et al., Proc. Natl. Acad. Sci. USA, 77, 4216-4220, 1980. The transfection of the vector DNA into CHO dhfr− cells may be carried out by methods known, for example, by the calcium phosphate method (described in F. L. Graham et al., Virology, 54, 536-539, 1973).

The above cells of the present invention, in which the expression vector was transfected, may be selected by utilizing the property that the transformant can grow even in a culture medium for selection in which the parent CHO *dhfr−* cells die for lack of thymidine and hypoxanthine.

The transformant in which the DHFR gene is amplified by the action of an antagonistic inhibitory agent for DHFR may be easily distinguished from that in which DHFR gene is not amplified, because the former can grow in a culture medium containing an antagonistic inhibitory agent for DHFR, e.g. methotrexate.

One of the transformants of the present invention obtained in this way, which was named CHO-B91 cells, has been deposited at the Institute for Fermentation, Osaka, Japan (IFO) with deposit No. IFO 50137 and deposited at the Fermentation Research Institute, Ibaraki, Japan (FRI) with deposit No. BP-1752.

The transformant of the present invention, which produces human IL4 efficiently and in quantity, may be cultured in a conventional culture medium (for example, $\alpha$-MEM containing 10%(v/v) fetal bovine serum) in the presence or absence of methotrexate. The desired protein, secreted into the culture medium, may be purified by conventional methods, for example salting-out techniques, ion-exchange chromatograpy, gel-filtration methods, hydrophobic column chromatograpy, affinity chromatography, etc.

The human IL4 obtained by the present invention improves the immune response and hence is useful for the treatment and prevention of diseases which require such improvement, for example cancer, infections, AIDS, functional immune deficiency, etc. The suitable dose may vary with the conditions of disease, age, sex and body weight, of the subject. For example, a suitable dosage is between 0.1 $\mu$g to 1 mg for intravenous administration.

The stimulatory activity on human lymphocyte growth was used as an indication of immune-activating effects of human IL4 in the present invention. The stimulatory activity on human lymphocyte growth was measured by the following method.

Lymphocytes prepared from human peripheral blood were suspended in the culture medium (RPMI-1640 containing 10%(v/v) fetal bovine serum and $5 \times 10^{-5}$ M 2-mercaptoethanol) containing phytohemagglutinin (10 $\mu$g/ml), and cultured for 4 to 6 days at 37°C in the presence of 5% $CO_2$. The cultured lymphocytes were washed with the culture medium (more than three times), and $5 \times 10^4$ lymphocytes thus obtained were again suspended in 200 $\mu$l of the culture medium. After adding a test sample therein, the suspension was further cultured for three days. It was pulsed with tritium labeled thymidine (0.25 $\mu$Ci/culture) for 12 hours before termination of culturing. Then, the incorporation of thymidine into the high molecular fraction were measured by a liquid scintillation counter. The relative activity measured was 2.5 ~ 10 $\times 10^5$ U/mg of protein.

Furthermore, antibody against human IL4 may be prepared by using human IL4 of the present invention as an antigen, and receptors of human IL4 can be isolated and purified by using the human IL4 as a ligand.

The following Examples illustrate the present invention in detail, but are in no way intended to limit the scope of the preent invention.

Example 1

Preparation of human IL4 cDNA

(i) Separation of mRNA for human IL4

Human peripheral blood lymphocytes were suspended in RPMI 1640 medium supplemented with 10%-(v/v) fetal bovine serum (hereafter "FBS"), $5 \times 10^{-5}$ M of 2-mercaptoethanol and 1 $\mu$g/ml of phorbol-12-mylistate-13-acetate (hereafter "PMA"), and then incubated at 37°C in 5% $CO_2$ for 12 hrs.

The cultured lymphocytes were washed with RPMI 1640 supplemented with 10%(v/v) FBS twice to remove PMA and resuspended in the same medium which contained 3 $\mu$g/ml of Concanavalin A (hereafter "Con-A") to induce IL-4. After 6 hrs induction, RNA was extracted from $6 \times 10^9$ of these lymphocytes using the guanidiumthiocyanate method (see Chivgwin, J.M., Przybyla, A.E., MacDonald, R.J. and Ruttev, W.J., Biochemistry, 18, 5294, 1979) and subsequently purified with an oligo(dT) cellulose column to yield 89 $\mu$g of mRNA containing poly $A^+ \cdot$ fraction.

An aliquot of mRNA obtained above was micro-injected into Xenopus laevis oocytes, which were incubated in modified Barth's medium (Colman, A., Transcription and Translation - a practical approach, pp 291; edited by Hames, B.D. and Higgins, S.J.; IRL Press 1984) at 20°C for 36 hr.

The biological activity for human IL4 produced threrein was examined by the activation human peripheral blood lymphocytes and human tonsilar B-lymphocytes as follows: human peripheral blood lymphocytes were cultured in RPMI-1640 supplemented with 10%(v/v) FBS, $5 \times 10^{-5}$ M of 2-mercaptoethanol and 10 $\mu$g/ml of phytohemagglutinin, at 37°C in 5% $CO_2$ for four to six days. These lymphocytes were washed (over three times) with medium and $5 \times 10^4$ cells were resuspended into 200 $\mu$l of medium which contained the sample to be examined. The incubation was continued for additional three days. During the final 12 hrs, the cells were pulse-labeled with [$^3$H]-thymidine (0.25 $\mu$Ci/culture) to measure the incorporation of the radioactivity into a high molecular weight fraction of the cells. Also, human lymphocytes prepared from human tonsils were purified using the rosetta formation using 2-aminoethylisothiouronium bromide treated sheep red blood cells (SRBC) to remove T lymphocytes. The purified B-lymphocytes obtained were incubated in RPMI-1640 supplemented with 10%(v/v) FBS, $5 \times 10^{-5}$ M of 2-mercaptoethanol and 5 $\mu$g/ml of anti-IgM antibody bound glass beads, at 37°C in 5% $CO_2$ for two to three days. Anti-IgM antibody bound beads were removed by density gradient centrifugation using lymphoprep (trade name, product of Nyegaard Inc.). B-lymphocytes were washed with the medium and then $5 \times 10^4$ cells were resuspended in 200 $\mu$l of medium each. Sample supernatants were added into this cell suspension and the mixture was allowed to incubate for the additional two days. The activity was measued by [$^3$H]-thymidine incorporation as described above.

(ii) Preparation of cDNA library

The cloning plasmid pCDV$_1$-PL (described in Reference A) was digested with the restriction enzyme KpnI. To the 3′-ends of this fragment, an oligo dT chain, average of 45 bases, was added with terminal deoxynucleotidyl transferase. This fragment was digested with EcoRI and the larger fragment was recovered from an agarose gel. Upon passing the larger fragment through an oligo (dA) column, the purified vector-primer was obtained.

Then, Plasmid pSP62-K2 (described in Reference A) was digested with SacI. To the 3′-ends of this fragment, an oligo dG chain, average of 14 bases, was added with terminal deoxynucleotidyl transferase. This fragment was digested with HindIII to obtain the linker DNA as the smaller fragment.

Using six $\mu$g of polyA$^+$ RNA described in Example 1(i), a cDNA was synthesized with 2 $\mu$g of vector primer as a template with 10 units of reverse-transcriptase. To the 3′-ends of the cDNA, an oligo dC chain, average of 14 bases, was added with terminal transferase. After the primer was digested with HindIII, the cDNA fragment was ligated with the linker. The first strand, RNA, was made into DNA with E. Coli polymerase I and R Nase H. E. Coli HB101 was transformed with the plasmid thus obtained to prepare a cDNA library which contained about one million independent clones.

(iii) Preparation of probe

Plasmid pSP6 KmIL4-374 (described in Reference A), which carried mouse IL4 cDNA, was digested with BamHI to excise an about 800 base pair insert. This fragment was further digested with Bal 31 in 40 $\mu$l of reaction mixture which contained 35 $\mu$g of DNA, 12 mM of $CaCl_2$, 12 mM of $MgCl_2$, 0.2 M of NaCl, 20 mM of Tris-HCl buffer (pH 8.0), 1 mM of EDTA and 0.3 units of Bal 31, at 30°C for 3 min. After the mixture was extracted the phenol, DNA was precipitated with ethanol, and then a BamHI linker was ligated with T4-ligase. The BamHI treated DNA fragment was inserted in the BamHI site of pUC 18 to prepare a plasmid pSP6mIL4Δ. The 5′-fragment obtained with BamHI/RsaI digestion does not contain any part of SP6 vector. At the same time, the RsaI/RsaI fragment was prepared, which covered most of the protein coding region.

(iv) Selection of cDNA clone

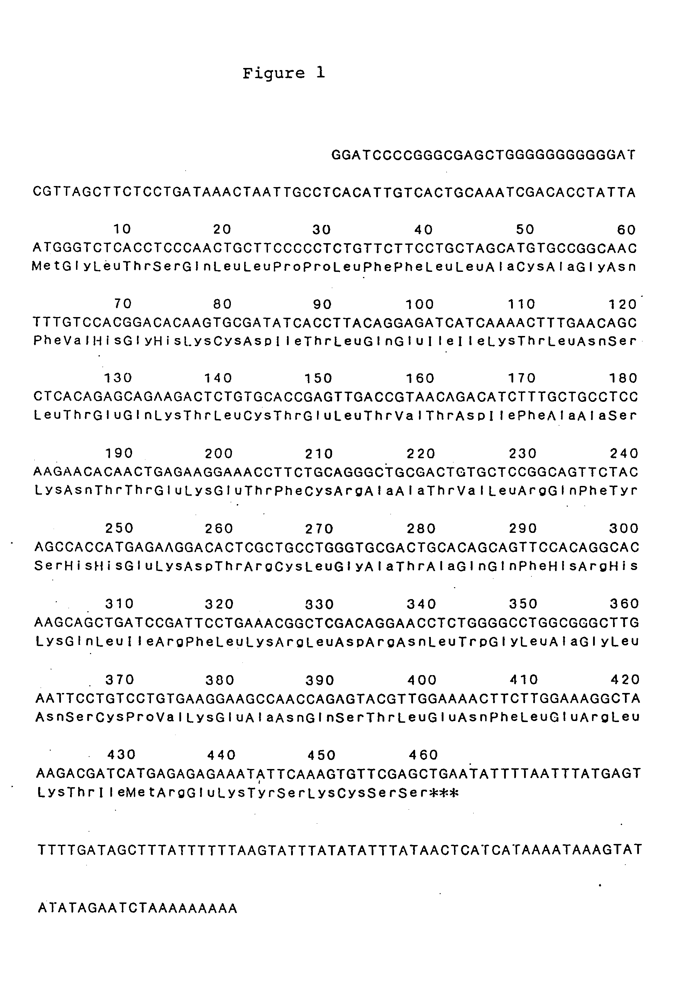

The cDNA library described above was adhered to GSN filters (product of German Science Inc.) to prepare four replica filters. Two fragments prepared from mouse IL4 cDNA, BamHI/RsaI (120 bp) and RsaI/RsaI (373 bp) were nick-translated independently to yield highly labeled probes (about 5 x 10$^8$ cpm/$\mu$g). Two replica filters were hybridized with each probe independently (50mM Tris-HCl buffer (pH 7.8), 10 mM EDTA, 1M NaCl, 0.1%(w/v) BSA, 0.1%(w/v) polyvinylpyrrolidone, 0.1%(w/v) Ficol 400, 0.1%(w/v) SDS and 50 $\mu$g/ml of sonicated salmon sperm DNA) at 37°C for 24hrs. All of the filters were washed in $6 \times$SSC ($1 \times$SSC: 0.15 M NaCl and 0.015M sodium citrate) and 0.1%(w/v) SDS at ambient temperature for 1 hr three times and then in $2 \times$SSC, 0.1%(w/v) SDS at 37°C for 20 min twice. One filter in each case was further washed in $2 \times$SSC, 0.1%(w/v) SDS at 42°C for 20 min. After autoradiography at -80°C, 258 and 68 clones showing a relatively strong signal compared with the background were selected from BamHI/RsaI and RsaI/RsaI hybridization, respectively. About ten colonies around one signal were picked and suspended in 100 $\mu$l of L-broth. Then the independent ten pools were combined to one pool to yield 26 and 7 pools from BamHI/RsaI and RsaI/RsaI hybridization, respectively. Plasmid DNA obtained from each pool was digested with SalI and the thus obtained templates were used to prepare 1.5 ~ 2.5 $\mu$g of mRNA with SP6 RNA polymerase. After phenol/chloroform treatment and ethanol precipitation, mRNA was dissolved in PBS to prepare a 0.3 $\mu g/\mu l$ solution. Each mRNA was micro-injected into Xenopus laevis Oocytes, (70 nl/Oocyte). Twenty Oocytes in each case was incubated in modified Barth's medium (10 $\mu l$/Oocyte) at 20°C for 30 hrs. and the supernatant was recovered to examine lymphocyte activation. As a result, two pools, #6 and 15 showed biological activity. E. Coli HB101 was transformed with the plasmid prepared from pool #6. Three hundred transformants were selected randomly, to prepare thirty new pools. The same screening was repeated for the new pools and five pools showed the activity again. Independent twenty clones were selected from two positive pools and the screening was repeated. Finally, single clones pSP(6-127) and pSP(6-230) were confirmed to yield IL4 activity. The nucleotide sequence was determined by the di-deoxy chain-termination method in both cases. As the result, two clones were obtained carrying essentially the same cDNA. The nucleotide sequence and the deduced amino acid sequence is shown in Figure 1.

Example 2

Construction of the expression vector for human IL4

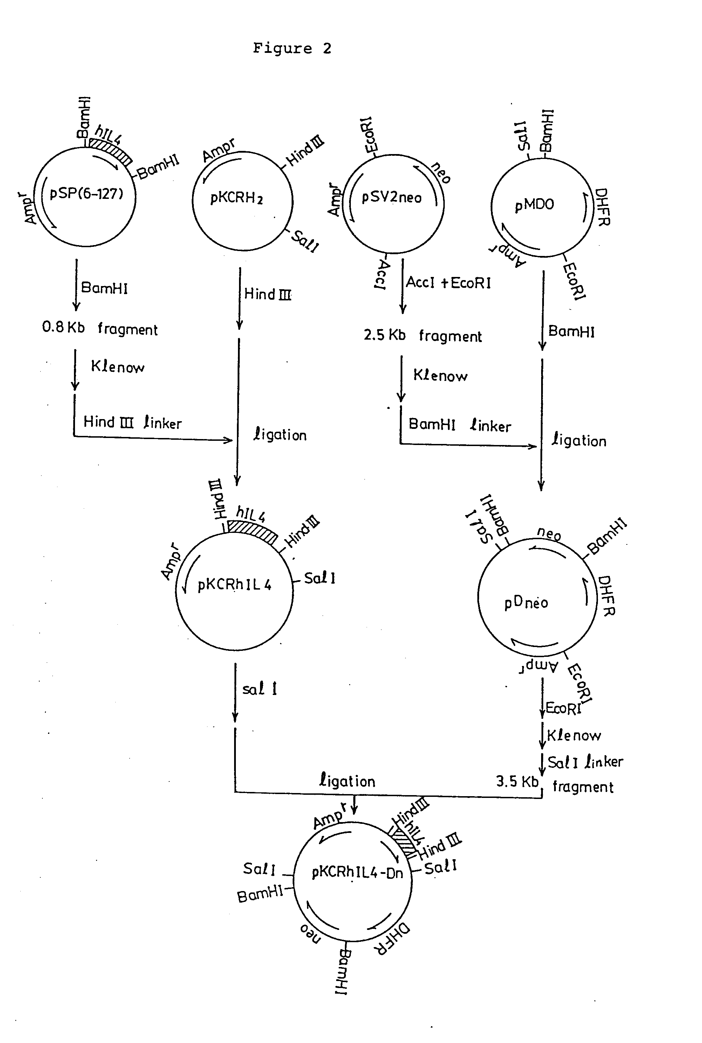

Plasmid pSP(6-127) was digested with BamHI to excise a 0.8 Kb fragment which contained the whole sequence of human IL4. After treatment with Klenow fragment and HindIII linker attachment, the DNA was inserted into HindIII site of pKCRH₂ (described in K. O'Hare et al., Proc. Natl. Acad. Sci. USA, 78, 1527-1531, 1981) to prepare plasmid pKCRhIL4 (see Figure 2).

Plasmid pSV2neo (described in R. C. Mulligan et al., Science, 209, 1422-1427, 1980) which can express the neomycin resistance gene neo in mammalian cells, was digested with AccI and EcoRI to prepare a 2.5 Kb fragment, which carried neo gene. The excised neo gene was treated with Klenow fragment and a BamHI linker attached. The DNA was then inserted in the BamHI site of plasmid pMDO which can express dihydrofolate reductase (described in S. K. Kim et al., Cell, 42, 129-138, 1985) to prepare plasmid pDneo (see Figure 2). In this plasmid, DHFR and neo are arranged in the same direction. pDneo was digested with EcoRI, treated with Klenow fragment and then a SalI linker attached . Upon digestion with SalI, a 6.5 Kb fragment purified was inserted into the SalI site of plasmid pKCRhIL4 to obtain the expression vector pKCRhIL4-Dn (see Figure 2).

Example 3

Construction of the expression vector of human IL4, wherein the sequence relating to the instability of mRNA was removed

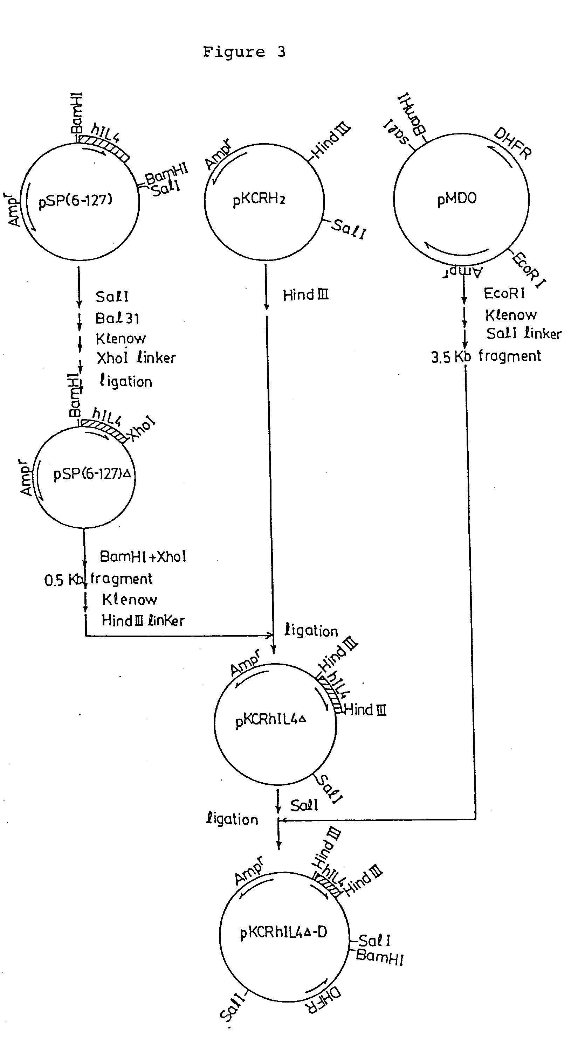

Plasmid pSP(6-127) was digested with SalI, and then further digested with 0.26 unites of Bal 31 (3.5 $\mu g$ DNA/40 $\mu l$ reaction mixture) at 30°C. Nine $\mu l$ of a sample solution was mixed in 1 $\mu l$ of 0.2M EGTA throughout the time course(2, 4, 6 and 10 minutes). An aliquot (2 $\mu l$) was digested with EcoRI and analyzed by PAGE. DNA of one pool which gave rise to a fragment around 100 bp (4 minutes reaction) was treated with Klenow fragment, ligated with an XhoI linker, recirculized and then incorporated to DH₁ to yield about 2,000 transformants. The plasmids were obtained from 120 independent clones and digested with EcoRI and XhoI to examine for the excision of the 100 bp fragment. Twenty-eight clones were selected and partially sequenced. One of them, plasmid pSP(6-127)Δ carried no 3′-noncoding sequence, whose TGA terminal codon and XhoI linker were directly ligated (see Figure 3). The fact shows that the entire nucleotide sequence downstream from the signal sequence for the termination of translation was removed.

Plasmid pSP(6-127)Δ was digested with BamHI and XhoI to excise a 0.5 Kb fragment. Upon treatment with Klenow fragment and attachment of a HindIII linker, the IL4 coding region was inserted into the HindIII site of plasmid pKCRH₂ to obtain plasmid pKCRhIL4Δ (see Figure 3).

Plasmid pMDO described in Example 2 was digested with EcoRI, treated with Klenow fragment, and a SalI linker attached. The 3.5 Kb of SalI fragment which contained DHFR gene was then inserted into the SalI site of plasmid pKCRhIL4Δ to obtain the plasmid pKCRhIL4Δ-D (see Figure 3).

9

Example 4

Preparation of transformant cells

The plasmid DNA, pKCRhIL4-Dn, was linearized by PvuI digestion. About 1 x 10⁶ CHO *dhfr⁻* cells (G. Urlaub et al., Proc. Natl. Acad. Sci. USA, 77, 4216-4220, 1980) in a 10 cm dish were transfected with 20 μg of the linearized DNA by the calcium phosphate co-precipitation method (F.L. Graham et al., Virology, 54, 536-539, 1973).

After 2 days of cultivation in complete medium (αMEM (Flow laboratories) containing 10%(v/v) FBS with hypoxanthine and thymidine), the cells were subcultured in selection medium (αMEM containing 10%(v/v) dialyzed FBS (GIBCO) without hypoxanthine and thymidine) at a split ratio of 1:5.

After 2 weeks of cultivation, a hundred of colonies out of about thousand were isolated and the production level of human IL4 for each clones were measured by growth stimulation of human lymphocytes as described above. The results of 10 representative clones, which were relatively well grown, are shown in Table II below.

Table II

| Clone No. | human IL4 activity (units/10⁶ cells/day) |
|-----------|------------------------------------------|
| A-1 | 4 |
| 2 | 4 |
| 3 | 3 |
| 4 | <1 |
| 5 | 4 |
| 6 | 4 |
| 7 | 5 |
| 8 | 10 |
| 9 | 4 |
| 10 | 7 |

CHO *dhfr⁻* cells were also transfected with the plasmid DNA, pKCRhIL4Δ-D, in the same manner and grown in selection medium for 2 weeks. A hundred colonies were isolated and the production level of human IL4 for each clones was determined. Table III shows the activities of 10 representative clones, which were relatively well grown.

Table III

| Clone No. | human IL4 activity (units/10⁶ cells/day) |
|-----------|------------------------------------------|
| B-1 | 50 |
| 2 | 80 |
| 3 | 270 |
| 4 | 165 |
| 5 | 120 |
| 6 | 95 |
| 7 | 10 |
| 8 | 50 |
| 9 | 330 |
| 10 | 240 |

Human IL4 production in the cells transfected with pKCRhIL4Δ-D was about 30 fold higher than that in the cells transfected with pKCRhIL4-Dn.

Two clones (clone No. A-8 and -10) from group A and 6 clones (clone No. B-1, -2, -3, -4, -9 and -10) from group B were selected and about 7×10⁵ cells of each clone were placed into a 10 cm disk and cultured in the selection medium containing 50 nM of methotrexate (hereafter "MTX") for 2 to 4 weeks. After the cells were grown to confluence, the IL4 activity in the medium was assayed as described above.

IL4 production in clones A-10 and B-1 was greatly enhanced after MTX treatment. 20 subclones were isolated from these clones.

Enhancement of IL4 production of clone B-9 was not observed by the treatment of 50 nM MTX. Thus, the cells were treated in the presence of 200 nM MTX in the same procedure, and 10 subclones were isolated.

The production of human IL4 in 15 representative clones derived from clones A-10, B-1 and B-9 were shown in Table IV below.

Table IV

| MTX resistant clone No. | MTX concentration (nM) | human IL4 activity (units/$10^6$ cells/day) |
|---|---|---|
| A-10-1 | 50 | 140 |
| A-10-2 | " | 250 |
| A-10-8 | " | 125 |
| A-10-13 | " | 170 |
| A-10-15 | " | 190 |
| B-1-4 | " | 1900 |
| B-1-5 | " | 2450 |
| B-1-6 | " | 2100 |
| B-1-10 | " | 2800 |
| B-1-14 | " | 1750 |
| B-9-1 | 200 | 5500 |
| B-9-2 | " | 3900 |
| B-9-3 | " | 5300 |
| B-9-4 | " | 2800 |
| B-9-5 | " | 3700 |

These 15 clones continued to produce IL4 in a high level during the subcultivation periods of 2 months. One of the high producer cell lines, B-9-1, was named as CHO-B91 and has been deposited at the Institute for Fermentation, Osaka, Japan (IFO) with deposit No. IFO 50137 and deposited at the Fermentation Research Institute, Ibaraki, Japan (FRI) with deposit No. BP-1752.

Example 5

Culturing CHO cells transfected by the human IL4 expression vector

Three grams of microcarrier beads (cytodex 3, Pharmacia) were suspended in phosphate buffered saline (pH 7.3) (hereafter "PBS(-)") and autoclaved in a 1 liter scale microcarrier flask (production by Bellco). Then, the PBS(-) was removed and 400 ml of serum free medium (SFM101, Nissui Seiyaku) containing 1%(v/v) fetal bovine serum (FBS), $7 \times 10^7$ CHO-B91 cells (prepared in Example 4) was added. The medium were stirred for 2 minutes at 20 r.p.m. and stood for 1 hour in 5% $CO_2$ in air at 37°C. The cells were attached to microcarrier beads after 4 cycles of this procedure.

Then, 100 ml of SFM 101 medium containing 1%(v/v) FBS were added into the flask and the cells were cultured for 2 days in 5% $CO_2$ in air at 37°C stirring at 20 r.p.m., 500 ml of the same medium were added and cultured for another 4 days.

The cells were grown in a monolayer on the surface of the microcarrier beads and washed with SFM101 medium to remove FBS.

The cells were maintained for another 24 days by feeding fresh medium and harvesting spent medium every day at 500 ml/day.

Example 6

Purification of human IL4

Twelve liters of the culture supernatants obtained in Example 5 were cooled to 4°C, and acidified by adding 120 ml of 10%(v/v) trifluoroacetic acid (hereafter "TFA"). After adding thereto 120g of Sepralyte C-1 (trade name, prepared by Analytichem International Co.) pre-washed with acetonitrile, the mixture was stirred slowly for two hours. The obtained mixture was allowed to stand for 30 minutes and the supernatants were removed therefrom to recover the beads on glass filter (3G). The recovered beads were washed with a total volume of two liters of 30%(v/v) acetonitrile (containing 0.1%(v/v) TFA) several times at 4°C, and then washed similarly with the total volume of 400 ml of 50%(v/v) acetonitrile (containing 0.1% TFA(v/v)) to wash out the absorbates. The obtained solution was concentrated under reduced pressure below 40°C to remove the acetonitrile, and was absorbed on cation-exchange column (Mono S, trade name, prepared by Pharmacia Co.) pre-equilibrated with 50 mM phosphate buffer (pH 6.0). The column was washed by adding 50 mM phosphate buffer (pH 6.0) for 10 minutes at a flow rate of 2 to 3 ml/min, and then washed by adding 50 mM phosphate buffer (pH 6.0) containing 0.3M sodium chloride until the absorbance (280 $\mu$m) become below 0.1. The desired substance was eluted with 50 mM phosphate buffer (pH 6.0) containing 0.4 M sodium chloride as eluent with monitoring by using a Phast System (trade name, prepared by Pharmacia Co.) to collect fractions having a molecular weight of 18 to 20 kd.

The collected fractions were diluted ten times with 50 mM phosphate buffer (pH 6.0) and again absorbed on cation-exchange column. The column was washed by adding 50 mM phosphate buffer (pH 6.0) containing 0.3 M sodium chloride until the absorbance (280 $\mu$m) became below 0.02, and then the NaCl concentration was increased linealy from 0.3 M to 0.5 M over 30 min. The fraction was also monitored with UV absorption and sodium dodecylsulfate/polyacrylamide gelelectrophoresis (hereafter "SDS/PAGE").

Ammonium sulfate was dissolved in the collected fractions at a final concentration of 1.7 M and the solution was absorbed on hydrophobic column chromatography (Phenyl•Superose, trade name, prepared by Pharmacia Co.) pre-equilibrated with 50 mM phosphate buffer (pH 6.0) containing 1.7 M ammonium sulfate, and then the concentration of ammonium sulfate was decreased linearly from 1.7 M to 0.0 M over 60 min. with monitoring by UV absorption and SDS/PAGE to collect fractions having a molecular weight of 18 to 20 kd. Mainly broad fractions at 0.5 to 0.3 M were obtained. Next, the fraction having a single band was collected and concentrated by using ultrafiltration (YM-10, trade name, prepared by Amicon Co.) The concentrate was separated by column chromatography with Superose 12 (2.6 x 80 cm) pre-equilibrated with 50 mM phosphate buffer (pH 6.0) to obtain a single broad peak around 0.74 of the column volume. This was concentrated by using ultrafiltration (YM-10) to obtain the final product.

The concentration of the final product was determined by the color method using bovine serum albumin as a standard (prepared by Biorad Co.). The yields were 5 mg. The recovery indicated by biological activity was 23% of the first culture supernatants.

Example 7

Molecular weight of human IL4

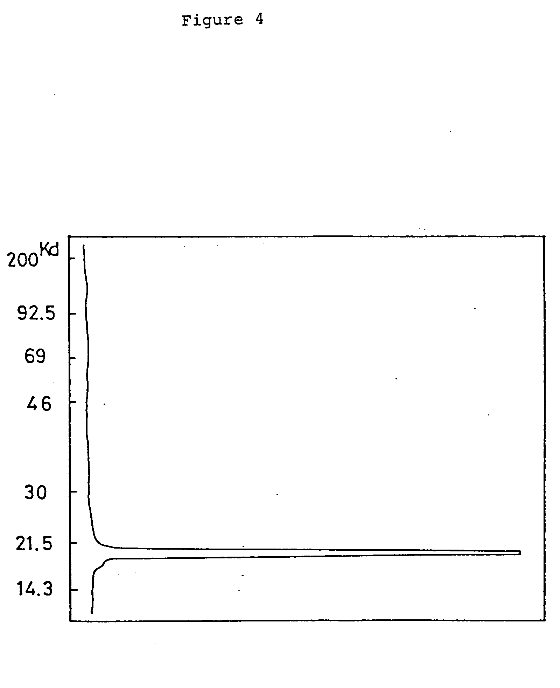

To the pure IL4 (2 $\mu$g) obtained in Example 6, the same amount of loading buffer (250 mM tris hydrochloric acid buffer (pH 6.8), 4%(w/v) SDS, 18%(v/v) glycerol, 1.4M 2-mercaptomethanol, 0.01%(w/v) bromophenol blue) was admixed. The mixture was heated for three minutes at 100°C, and then separated by using SDS/PAGE [10 ~ 20%, 1 mm, slubgel, product of Dai-ichi-Kagaku-Yakuhin]. A single band was obserbed by Coomasie Blue staining at 18 kd. This band was reconfirmed by Silver-stain and the purity was measured as over 98% by a densitometric scan (CS-910, Shimazu) (see Figure 4).

Example 8

Analysis of N-terminal amino acid sequence in human IL4

The following single sequence was confirmed by using a liquid phase protein sequencer (Model 477A, prepared by Applied Biosystem Co.):

His-Lys-X-Asp-Ile-Thr-Leu-Gln-Glu-Ile-Ile

-Lys-Thr-Leu-Asn-Ser-Leu-Thr-Glu-Gln-Lys

-Thr-Leu-X-Thr-Glu-Leu

(wherein X corresponds to Cys).

The above N-terminal sequence is completely identical with that of human IL4 obtained by culturing COS-7 cells [see Proc. Natl. Acad. Sci., 83, 5394 (1986)]. The above result shows that the separation of a signal peptide between the 24th amino acid, glycine and the 25th amino acid, histidine, both deduced from cDNA sequence, was carried out completely.

Example 9

Amino acid components in human IL4

100 $\mu$g of the sample were dissolved in 6N hydrochloric acid and 4%(w/v) thioglycollic acid and hydrolyzed over 22 hours at 110°C under reduced pressure. The hydrolyzate was analyzed by using an amino acid analyzer (Model 835, prepared by Hitachi Co.). The results are shown in Table V below.

Table V

| Amino Acids Analysis | | |
|---|---|---|
| | calculated | observed |
| aspartic acid and asparagine | 10 | 10.3 |
| threonine | 15 | 14.5 |
| serine | 8 | 7.5 |
| glutamic acid and glutamine | 17 | 17.0 |
| glycine | 3 | 3.3 |
| alanine | 8 | 8.5 |
| valine | 3 | 3.2 |
| cystein | 6 | 5.3 |
| methionine | 1 | 1.0 |
| isoleucine | 6 | 5.5 |
| leucine | 16 | 16.4 |
| tyrosine | 2 | 2.1 |
| phenylalanine | 6 | 6.2 |
| lysine | 12 | 12.4 |
| histidine | 5 | 5.2 |
| arginine | 9 | 9.0 |
| proline | 1 | 1.1 |
| tryptophane | 1 | 0.6 |
| total amino acids | 129 | |

Example 10

Sugar Content of human IL4

The molecular weight of only the protein moiety deduced from the cDNA is 14962. Hence, the difference in molecular weight, i.e. about 3000 (about 17%) is considered to be the molecular weight of the sugar chain, which means the sugar content.

### Example 11

#### Sugar components of human IL4

(a) neutral sugar

To 30 $\mu$g of a sample was added 2N trifluoroacetic acid, and the mixture was heated for 6 hours at 100°C to hydrolyze such. Then, the sample was concentrated under reduced pressure to dryness. The obtained solid was dissolved in distilled water to perform HPLC analysis.

The analysis was carried out by using a TSK-gel sugar AXG column (prepared by Toyo Jozo Co.) in 0.5M borate buffer (pH 8.7). As a result, it was confirmed that the sample comprises mannose, fucose and galactose.

(b) amino sugar

To a sample was added 4N hydrochloric acid and the subsequent procedures were carried out similarly to that above for neutral sugar to obtain the sample for analysis.

HPLC analysis was carried out by using a TSK-gel SCX column (prepared by Toyo Jozo Co.) in 0.16M borate buffer (pH 7.6). As a result, it was confirmed that the sample comprises glucosamine.

(c) sialic acid

To a sample was added 0.01M sulfuric acid, and the mixture was heated for one hour at 80°C to hydrolyze such. Then, the sample was concentrated under reduced pressure to dryness. The obtained solid was dissolved in distilled water to perform HPLC analysis.

The analysis was carried out by using a Gelpak C-620-10 (H$^+$ form) column (prepared by Hitachi Co.) in 0.3% phosphoric acid. As a result, it was confirmed that the sample comprises N-acetylneuraminic acid (hereafter "NANA").

Further, the ratios of each of the sugar components is shown in Teble VI below.

Table VI

| Mol of sugar components/total mols of sugar | |
|---|---|
| mannose | 3.52 |
| fucose | 1.42 |
| galactose | 3.01 |
| glucosamine | 5.06 |
| NANA | 2.06 |

### Example 12

#### Isolectric point of human IL4

Chromatofocusing using a Mono P column (HR 5/20, prepared by Pharmacia Co.) was performed by using 0.2 mg of a sample.

25 mM triethylamine buffer (pH 11.0) as a starting condition was changed to 1/45 diluted pharmalyte 8-10.3 (pH 8.0, product of Pharmacia). A single peak was eluted at pH 9.96 so that the isoelectric point is about pH 10.

Example 13

Deglycosylation of human IL4

Five μg of a sample (1 mg/ml) were treated at 100°C for 3 minutes with SDS at a final concentration of 0.5%(w/v) and 0.1M 2-mercaptoethanol and then reacted for 16 hours at 37°C together with sodium phosphate buffer (pH 8.6) at a final concentration of 0.2M, 10 mM 1,10-phenanthroline, 1.25%(v/v) Nonidet P-40 (NP-40), 10 units/ml N-glycanase (prepared by Genzyme Co.). An aliquot was separated by SDS/PAGE (20%, 1 mm slubgel). A single band was observed Coomasie blue staining at 15 kd.

While the invention has been described in detail and with reference to specific embodiments thereof, it will be apparent to one skilled in the art that changes and modifications can be made therein without departing from the spirit and scope thereof.

**Claims**

**Claims for the following Contracting States : AT, BE, CH, DE, FR, GB, IT, LI, LU, NL, SE**

1. Human interleukin 4, characterized by
    (a) a total molecular weight of 17000 to 19000, and
    (b) a sugar content thereof corresponding to a molecular weight of 2000 to 4000.

2. The human interleukin 4 according to claim 1, wherein the sugar content thereof is corresponding to a molecular weight of 2500 to 3500.

3. The human interleukin 4 according to claim 1 or 2, further characterized by
    (c) an isoelectric point of 8,5 to 10,5.

4. The human interleukin 4 according to claim 1 or 2, wherein the isoelectric point is 9,8 to 10,1.

5. The human interleukin 4 according to any of claims 1 to 4, further characterized by
    (d) the sugar content thereof comprising at least mannose, fucose and galactose as neutral sugars.

6. The human interleukin 4 according to any of claims 1 to 4, further characterized by
    (e) the sugar content thereof comprising at least glucosamine as an amino sugar.

7. The human interleukin 4 according to any of claims 1 to 4, further characterized by
    (f) the sugar content thereof comprising at least N-acetylneuraminic acid as a sialic acid.

8. The human interleukin 4 according to any of claims 1 to 4, further characterized by
    (d) the sugar content thereof comprising at least mannose, fucose and galactose as neutral sugars,
    (e) the sugar content thereof comprising at least glucosamine as an amino sugar, and
    (f) the sugar content thereof comprising at least N-acetylneuraminic acid as a sialic acid.

9. The human interleukin 4 according to claim 8, further characterized by
    (g) mannose, fucose, galactose, glucosamine and N-acetylneuraminic acid being contained in amounts of 3.0 to 4.0, 1.0 to 2.0, 2.5 to 3.5, 4.5 to 5.5 and 1.5 to 2.5, mols per total mols of sugar, respectively.

10. The human interleukin 4 according to claim 1, which is produced by using as a host, a transformant which is obtainable by transfecting dihydrofolate reductase-defective Chinese hamster ovary cells with an expression vector for human interleukin 4, wherein the expression vector comprises:
    (i) a DNA sequence encoding human interleukin 4 at a position downstream from an SV40 early promoter, and
    (ii) a DNA sequence encoding dihydrofolate reductase at a position downstream from a promoter operating in eucaryotic cells.

11. The human interleukin 4 according to claim 10, wherein in the expression vector for human interleukin 4, at least the human interleukin 4 gene nucleotide sequence for instability is removed.

**12.** The human interleukin 4 according to claim 10 or 11, further characterized by
(c) an isoelectric point of 8,5 to 10,5.

**13.** The human interleukin 4 according to any of claims 10 to 12, further characterized by
(d) the sugar content thereof comprising at least mannose, fucose and galactose as neutral sugars.

**14.** The human interleukin 4 according to any of claims 10 to 12, further characterized by
(e) the sugar content thereof comprising at least glucosamine as an amino sugar.

**15.** The human interleukin 4 according to any of claims 10 to 12, further characterized by
(f) the sugar content thereof comprising at least N-acetylneuraminic acid as a sialic acid.

**16.** The human interleukin 4 according to any of claims 10 to 12, further characterized by
(d) the sugar content thereof comprising at least mannose, fucose and galactose as neutral sugars,
(e) the sugar content thereof comprising at least glucosamine as an amino sugar, and
(f) the sugar content thereof comprising at least N-acetylneuraminic acid as a sialic acid.

**17.** The human interleukin 4 according to claim 16, further characterized by
(g) mannose, fucose, galactose, glucosamine and N-acetylneuraminic acid being contained in amounts of 3.0 to 4.0, 1.0 to 2.0, 2.5 to 3.5, 4.5 to 5.5 and 1.5 to 2.5, mols per total mols of sugar, respectively.

**18.** An expression vector for human interleukin 4, characterized by
(a) a total molecular weight of 17,000 to 19,000, and
(b) a sugar content thereof corresponding to a molecular weight of 2,000 to 4,000,
which comprises:
(i) a DNA sequence encoding human interleukin 4 at a position downstream from an SV40 early promoter, and
(ii) a DNA sequence encoding dihydrofolate reductase at a position downstream from a promoter operating in eucaryotic cells.

**19.** The expression vector according to claim 18, wherein at least the human interleukin 4 gene nucleotide sequence for instability is removed.

**20.** A transformant which is obtainable by transfecting dihydrofolate reductase-defective Chinese hamster ovary cells with an expression vector for human interleukin 4, characterized by
(a) a total molecular weight of 17,000 to 19,000, and (b) a sugar content thereof corresponding to a molecular weight of 2,000 to 4,000,
which comprises:
(i) a DNA sequence encoding human interleukin 4 at a position downstream from an SV40 early promoter, and
(ii) a DNA sequence encoding dihydrofolate reductase at a position downstream from a promoter operating in eucaryotic cells.

**21.** The transformant according to claim 20, wherein in the expression vector for human interleukin 4, at least the human interleukin 4 gene nucleotide sequence for instability is removed.

**Claim for the following Contracting States : ES, GR**

1. A process for the production of human interleukin 4 characterized by
(a) a total molecular weight of 17000 to 19000, and
(b) a sugar content thereof corresponding to a molecular weight of 2000 to 4000,
which is characterized by culturing a transformant obtained by transfecting dihydrofolate reductase-defective Chinese hamster ovary cells with an expression vector for human interleukin 4 which comprises:
(i) a DNA sequence encoding human interleukin 4 at a position downstream from an SV40 early promoter and

(ii) a DNA sequence encoding dihydrofolate reductase at a position downstream from a promotor operating in eukaryotic cells.

**Patentansprüche**

**Patentansprüche für folgende Vertragsstaaten : AT, BE, CH, DE, FR, GB, IT, LI, LU, NL, SE**

1.  Humaninterleukin 4, gekennzeichnet durch
    (a) ein Gesamtmolekulargewicht von 17 000 bis 19 000 und
    (b) einen Zuckergehalt enstsprechend einem Molekulargewicht von 2 000 bis 4 000.

2.  Humaninterleukin 4 nach Anspruch 1, wobei sein Zukkergehalt einem Molekulargewicht von 2 500 bis 3 500 entspricht.

3.  Humaninterleukin 4, nach Anspruch 1 oder 2, weiterhin gekennzeichnet durch
    (a) einen isoelektrischen Punkt von 8,5 bis 10,5.

4.  Humaninterleukin 4 nach Anspruch 1 oder 2, wobei der isoelektrische Punkt 9,8 bis 10,1 beträgt.

5.  Humaninterleukin 4 nach einem der Ansprüche 1 bis 4, weiterhin dadurch gekennzeichnet, daß
    (d) sein Zuckeranteil mindestens Mannose, Fucose und Galactose als Neutralzucker umfaßt.

6.  Humaninterleukin 4 nach einem der Ansprüche 1 bis 4, weiterhin dadurch gekennzeichnet, daß
    (e) sein Zuckeranteil mindestens Glucosamin als Aminozucker umfaßt.

7.  Humaninterleukin 4 nach einem der Ansprüche 1 bis 4, weiterhin dadurch gekennzeichnet, daß
    (f) sein Zuckeranteil mindestens N-Acetylneuraminsäure als eine Sialsäure umfaßt.

8.  Humaninterleukin 4 nach einem der Ansprüche 1 bis 4, weiterhin gekennzeichnet dadurch, daß
    (d) sein Zuckeranteil mindestens Mannose, Fucose und Galactose als Neutralzucker,
    (e) sein Zuckeranteil mindestens Glucosamin als Aminozucker und
    (f) sein Zuckeranteil mindestens N-Acetylneuraminsäure als eine Sialsäure umfaßt.

9.  Humaninterleukin 4 nach Anspruch 8, weiterhin gekennzeichnet durch
    (g) die Anwesenheit von Mannose, Fucose, Galactose, Glucosamin bzw. N-Acetylneuraminsäure in Mengen von 3,0 bis 4,0, 1,0 bis 2,0, 2,5 bis 3,5, 4,5 bis 5,5 bzw. 1,5 bis 2,5 Mol(en) pro Gesamtmolmenge Zucker.

10. Humaninterleukin 4 nach Anspruch 1, hergestellt unter Verwendung eines Transformanten, der durch Transfektion von Eierstockzellen des Chinesischen Hamsters mit Dihydrofolatreduktasedefekt mit einem Expressionsvektor für Humaninterleukin 4 erhältlich ist, als Wirt, wobei der Expressionsvektor
    (I) eine DNA-Sequenz mit Codierung für Humaninterleukin 4 an einer Stelle stromabwärts von einem SV40 Frühpromotor und
    (II) eine DNA-Sequenz mit Codierung für Dihydrofolatreduktase an einer Stelle stromabwärts von einem in eukaryotischen Zellen operierenden Promotor umfaßt.

11. Humaninterleukin 4 nach Anspruch 10, wobei in dem Expressionsvektor für Humaninterleukin 4 mindestens die Humaninterleukin-4-Gennucleotidsequenz für Instabilität entfernt ist.

12. Humaninterleukin 4 nach Anspruch 10 oder 11, weiterhin gekennzeichnet durch
    (c) einen isoelektrischen Punkt von 8,5 bis 10,5.

13. Humaninterleukin 4 nach einem der Ansprüche 10 bis 12, weiterhin dadurch gekennzeichnet, daß
    (d) sein Zuckeranteil mindestens Mannose, Fucose und Galactose als Neutralzucker umfaßt.

14. Humaninterleukin 4 nach einem der Ansprüche 10 bis 12, weiterhin dadurch gekennzeichnet, daß
    (e) sein Zuckeranteil mindestens Glucosamin als Aminozucker umfaßt.

15. Humaninterleukin 4 nach einem der Ansprüche 10 bis 12, weiterhin dadurch gekennzeichnet, daß

(f) sein Zuckeranteil mindestens N-Acetylneuraminsäure als eine Sialsäure umfaßt.

16. Humaninterleukin 4 nach einem der Ansprüche 10 bis 12, weiterhin dadurch gekennzeichnet, daß

(d) sein Zuckeranteil mindestens Mannose, Fucose und Galactose als Neutralzucker,

(e) sein Zuckeranteil mindestens Glucosamin als Aminozucker und

(f) sein Zuckeranteil mindestens N-Acetylneuraminsäure als eine Sialsäure umfaßt.

17. Humaninterleukin 4 nach Anspruch 16, weiterhin gekennzeichnet durch

(g) die Anwesenheit von Mannose, Fucose, Galactose, Glucosamin bzw. N-Acetylneuraminsäure in Mengen von 3,0 bis 4,0, 1,0 bis 2,0, 2,5 bis 3,5, 4,5 bis 5,5 bzw. 1,5 bis 2,5 Mol(en) pro Gesamtmolmenge Zucker.

18. Expressionsvektor für Humaninterleukin 4, gekennzeichnet durch

(a) ein Gesamtmolekulargewicht von 17 000 bis 19 000 und

(b) seinen Zuckergehalt enstsprechend einem Molekulargewicht von 2 000 bis 4 000, umfassend

(I) eine DNA-Sequenz mit Codierung für Humaninterleukin 4 an einer Stelle stromabwärts von einem SV40 Frühpromotor und

(II) eine DNA-Sequenz mit Codierung für Dihydrofolatreduktase an einer Stelle stromabwärts von einem in eukaryotischen Zellen operierenden Promotor.

19. Expressionsvektor nach Anspruch 18, wobei mindestens die Humaninterleukin-4-Gennucleotidsequenz für Instabilität entfernt ist.

20. Transformant, der durch Transfektion von Eierstockzellen des Chinesischen Hamsters mit Dihydrofolatreduktasedefekt mit einem Expressionsvektor für Humaninterleukin 4 erhältlich ist, gekennzeichnet durch

(a) ein Gesamtmolekulargewicht von 17 000 bis 19 000 und

(b) seinen Zuckergehalt enstsprechend einem Molekulargewicht von 2 000 bis 4 000, umfassend

(I) eine DNA-Sequenz mit Codierung für Humaninterleukin 4 an einer Stelle stromabwärts von einem SV40 Frühpromotor und

(II) eine DNA-Sequenz mit Codierung für Dihydrofolatreduktase an einer Stelle stromabwärts von einem in eukaryotischen Zellen operierenden Promotor.

21. Transformant nach Anspruch 20, wobei in dem Expressionsvektor für Humaninterleukin 4 mindestens die Humaninterleukin-4-Gennucleotidsequenz für Instabilität entfernt ist.

**Patentanspruch für folgednde Vertragsstaaten : ES, GR**

1. Verfahren zur Herstellung eines durch

(a) ein Gesamtmolekulargewicht von 17 000 bis 18 000 und

(b) seinen Zuckergehalt entsprechend einem Molekulargewicht von 2 000 bis 4 000 gekennzeichneten Humaninterleukins 4, dadurch gekennzeichnet, daß ein durch Transfektion von Eierstockzellen des Chinesischen Hamsters mit Dihydrofolatreduktasedefekt mit einem Expressionsvektor für Humaninterleukin 4 erhaltener Transformant gezüchtet wird, wobei der Expressionsvektor

(I) eine DNA-Sequenz mit Codierung für Humaninterleukin 4 an einer Stelle stromabwärts von einem SV40 Frühpromotor und

(II) eine DNA-Sequenz mit Codierung für Dihydrofolatreduktase an einer Stelle stromabwärts von einem in eukaryotischen Zellen operierenden Promotor umfaßt.

**Revendications**

**Revendications pour les Etats contractants suivants : AT, BE, CH, DE, FR, GB, IT, LI, LU, NL, SE**

1. Interleukine 4 humaine, caractérisée par

(a) un poids moléculaire total compris entre 17 000 et 19 000 et

(b) sa teneur en sucres correspondant à un poids moléculaire compris entre 2 000 et 4 000.

2. Interleukine 4 humaine selon la revendication 1, dont la teneur en sucres correspond à un poids moléculaire compris entre 2 500 et 3 500.

3. Interleukine 4 humaine selon la revendication 1 ou 2, caractérisée en outre par
   (c) un point isoélectrique compris entre 8,5 et 10,5.

4. Interleukine 4 humaine selon la revendication 1 ou 2, dont le point isoélectrique est compris entre 9,8 et 10,1.

5. Interleukine 4 humaine selon l'une quelconque des revendications 1 à 4, caractérisée en outre par
   (d) sa teneur en sucres comprenant au moins du mannose, du fucose et du galactose comme sucres neutres.

6. Interleukine 4 humaine selon l'une quelconque des revendications 1 à 4, caractérisée en outre par
   (e) sa teneur en sucres comprenant au moins de la glucosamine comme sucre aminé.

7. Interleukine 4 humaine selon l'une quelconque des revendications 1 à 4, caractérisée en outre par
   (f) sa teneur en sucres comprenant au moins de l'acide N-acétylneuraminique comme acide sialique.

8. Interleukine 4 humaine selon l'une quelconque des revendications 1 à 4, caractérisée en outre par
   (d) sa teneur en sucres comprenant au moins du mannose, du fucose et du galactose comme sucres neutres,
   (e) sa teneur en sucres comprenant au moins de la glucosamine comme sucre aminé et
   (f) sa teneur en sucres comprenant au moins de l'acide N-acétylneuraminique comme acide sialique.

9. Interleukine 4 humaine selon la revendication 8, caractérisée en outre par
   (g) le fait que le mannose, le fucose, le galactose, la glucosamine et l'acide N-acétylneuraminique sont contenus en quantités comprises respectivement entre 3,0 et 4,0, 1,0 et 2,0, 2,5 et 3,5, 4,5 et 5,5 et 1,5 et 2,5 moles par moles totales de sucre.

10. Interleukine 4 humaine selon la revendication 1, laquelle est produite par emploi comme hôte d'un transformant qui peut être obtenu par transfection de cellules ovariennes de hamster chinois dépourvues de dihydrofolate-réductase à l'aide d'un vecteur d'expression de l'interleukine 4 humaine, le vecteur d'expression comprenant :
    (i) une séquence d'ADN codant pour l'interleukine 4 humaine à une position en aval d'un promoteur précoce SV40, et
    (ii) une séquence d'ADN codant pour la dihydrofolate-réductase à une position en aval d'un promoteur opérant dans des cellules eucaryotes.

11. Interleukine 4 humaine selon la revendication 10, dans le vecteur d'expression de l'interleukine 4 humaine, au moins la séquence de nucléotides du gène de l'interleukine 4 humaine pour l'instabilité étant retirée.

12. Interleukine 4 humaine selon la revendication 10 ou 11, caractérisée en outre par
    (c) un point isoélectrique compris entre 8,5 et 10,5.

13. Interleukine 4 humaine selon l'une quelconque des revendications 10 à 12, caractérisée en outre par
    (d) sa teneur en sucres comprenant au moins du mannose, du fucose et du galactose comme sucres neutres.

14. Interleukine 4 humaine selon l'une quelconque des revendications 10 à 12, caractérisée en outre par
    (e) sa teneur en sucres comprenant au moins de la glucosamine comme sucre aminé.

15. Interleukine 4 humaine selon l'une quelconque des revendications 10 à 12, caractérisée en outre par
    (f) sa teneur en sucres comprenant au moins de l'acide N-acétylneuraminique comme acide sialique.

16. Interleukine 4 humaine selon l'une quelconque des revendications 10 à 12, caractérisée en outre par
    (d) sa teneur en sucres comprenant au moins du mannose, du fucose et du galactose comme sucre neutres,
    (e) sa teneur en sucres comprenant au moins de la glucosamine comme sucre aminé et
    (f) sa teneur en sucres comprenant au moins de l'acide N-acétylneuraminique comme acide sialique.

**17.** Interleukine 4 humaine selon la revendication 16, caractérisée en outre par

(g) le fait que le mannose, le fucose, le galactose, la glucosamine et l'acide N-acétylneuraminique sont contenus en des quantités comprises respectivement entre 3,0 et 4,0, 1,0 et 2,0, 2,5 et 3,5, 4,5 et 5,5 et 1,5 et 2,5 moles par moles totales de sucre.

**18.** Vecteur d'expression de l'interleukine 4 humaine caractérisée par

(a) un poids moléculaire total compris entre 17 000 et 19 000, et

(b) sa teneur en sucres correspondant à un poids moléculaire compris entre 2 000 et 4 000, lequel comprend :

(i) une séquence d'ADN codant pour l'interleukine 4 humaine à une position en aval d'un promoteur précoce SV40 et

(ii) une séquence d'ADN codant pour la dihydrofolate-réductase à une position en aval d'un promoteur opérant dans des cellules eucaryotes.

**19.** Vecteur d'expression selon la revendication 18, dans lequel on a retiré au moins la séquence de nucléotides du gène de l'interleukine 4 humaine pour l'instabilité.

**20.** Transformant qu'il est possible d'obtenir par transfection de cellules ovariennes de hamster chinois dépourvues de dihydrofolate-réductatse à l'aide d'un vecteur d'expression de l'interleukine 4 humaine caractérisée par

(a) un poids moléculaire total compris entre 17 000 et 19 000 et

(b) sa teneur en sucres correspondant à un poids moléculaire compris entre 2 000 et 4 000, lequel comprend :

(i) une séquence d'ADN codant pour l'interleukine 4 humaine à une position en aval d'un promoteur précoce SV40, et

(ii) une séquence d'ADN codant pour la dihydrofolate-réductase à une position en aval d'un promoteur opérant dans des cellules eucaryotes.

**21.** Transformant selon la revendication 20, dans lequel on a retiré dans le vecteur d'expression de l'interleukine 4 humaine, au moins la séquence de nucléotides du gène de l'interleukine 4 humaine pour l'instabilité.

**Revendication pour les Etats contractants suivants : ES, GR**

**1.** Procédé de production d'interleukine 4 humaine, caractérisée par

(a) un poids moléculaire total compris entre 17 000 et 19 000 et

(b) sa teneur en sucres correspondant à un poids moléculaire compris entre 2 000 et 4 000,

lequel est caractérisé par la culture d'un transformant obtenu par transfection de cellules ovariennes de hamster chinois dépourvues de dihydrofolate-réductase à l'aide d'un vecteur d'expression de l'interleukine 4 humaine qui comprend :

(i) une séquence d'ADN codant pour l'interleukine 4 humaine à une position en aval d'un promoteur précoce SV40 et

(ii) une séquence d'ADN codant pour la dihydrofolate-réductase à une position en aval d'un promoteur opérant dans des cellules eucaryotes.

Figure 1

GGATCCCCGGGCGAGCTGGGGGGGGGGGAT

CGTTAGCTTCTCCTGATAAACTAATTGCCTCACATTGTCACTGCAAATCGACACCTATTA

```
        10        20        30        40        50        60
ATGGGTCTCACCTCCCAACTGCTTCCCCCTCTGTTCTTCCTGCTAGCATGTGCCGGCAAC
MetGlyLeuThrSerGlnLeuLeuProProLeuPhePheLeuLeuAlaCysAlaGlyAsn

        70        80        90       100       110       120
TTTGTCCACGGACACAAGTGCGATATCACCTTACAGGAGATCATCAAAACTTTGAACAGC
PheValHisGlyHisLysCysAspIleThrLeuGlnGluIleIleLysThrLeuAsnSer

       130       140       150       160       170       180
CTCACAGAGCAGAAGACTCTGTGCACCGAGTTGACCGTAACAGACATCTTTGCTGCCTCC
LeuThrGluGlnLysThrLeuCysThrGluLeuThrValThrAspIlePheAlaAlaSer

       190       200       210       220       230       240
AAGAACACAACTGAGAAGGAAACCTTCTGCAGGGCTGCGACTGTGCTCCGGCAGTTCTAC
LysAsnThrThrGluLysGluThrPheCysArgAlaAlaThrValLeuArgGlnPheTyr

       250       260       270       280       290       300
AGCCACCATGAGAAGGACACTCGCTGCCTGGGTGCGACTGCACAGCAGTTCCACAGGCAC
SerHisHisGluLysAspThrArgCysLeuGlyAlaThrAlaGlnGlnPheHisArgHis

       310       320       330       340       350       360
AAGCAGCTGATCCGATTCCTGAAACGGCTCGACAGGAACCTCTGGGGCCTGGCGGGCTTG
LysGlnLeuIleArgPheLeuLysArgLeuAspArgAsnLeuTrpGlyLeuAlaGlyLeu

       370       380       390       400       410       420
AATTCCTGTCCTGTGAAGGAAGCCAACCAGAGTACGTTGGAAAACTTCTTGGAAAGGCTA
AsnSerCysProValLysGluAlaAsnGlnSerThrLeuGluAsnPheLeuGluArgLeu

       430       440       450       460
AAGACGATCATGAGAGAGAAATATTCAAAGTGTTCGAGCTGAATATTTTAATTTATGAGT
LysThrIleMetArgGluLysTyrSerLysCysSerSer***
```

TTTTGATAGCTTTATTTTTTAAGTATTTATATATTTATAACTCATCATAAAATAAAGTAT

ATATAGAATCTAAAAAAAAAA

Figure 2

Figure 3

Figure 4